# EUROPEAN PATENT SPECIFICATION

(11) **EP 4 637 889 B1**
(45) Date of publication and mention of the grant of the patent: **09.12.2026**
(21) Application number: 23828780.9
(22) Date of filing: 18.12.2023
(51) Int. Cl.: A61M 16/06

(54) **MEDICAL FACE MASK**
MEDIZINISCHE GESICHTSMASKE
MASQUE FACIAL MÉDICAL

(30) Priority: 20.12.2022 GB 202219290
(43) Date of publication of application: 29.10.2025
(73) Proprietor: Sallow Pightle Ltd, Ashington, Sussex RH20 3LF (GB)
(72) Inventor: STANDLEY, Thomas David Auger, Ashington Sussex RH20 3LF (GB)
(74) Representative: McDougall, James
(86) International application number: PCT/GB2023/053281
(87) International publication number: WO 2024/134168

(56) References cited:
- WO-A1-2021/035306
- WO-A1-2022/099377
- CN-U- 216 366 212

## Description

### Field

This specification relates to an apparatus for delivering gas to a user and in particular to a medical face mask with gas connector.

### Background

Numerous prior art devices exist for administering medical gases to a patient without the application of positive pressure to the patient airway. These can be classified as closed devices which have no direct communication to the surrounding atmosphere and semi-open devices where direct communication(s) to the surrounding atmosphere exist. Examples of closed devices include a mouthpiece either connected to a closed breathing system of adequate volume or used with a demand valve connected to a pressurised gas source, or by replacing the environment surrounding the patient completely with the gas that is to be inhaled (e.g. using a hood). Successful use of mouthpieces requires the co-operation of the user; hoods require high gas flows to maintain accuracy of administration which raises costs.

Current semi-open devices for delivering supplemental oxygen to a patient work by increasing the volume of oxygen available to the patient over and above that which is ordinarily present in atmospheric air. Any deficiency in terms of administered total gas volume compared to the volume the user breathes in will be taken from the surrounding atmospheric air (e.g. through vents between the mask and surrounding atmosphere). As a result, the volume of oxygen inhaled by the patient will be variably mixed with atmospheric air, with both device factors (e.g. leak area, device volume, vent resistance, oxygen flow) and patient factors (e.g. inspiratory flow, length of expiratory pause) affecting the degree of dilution of the fresh gas flow going into the device. For example, when a current industry standard high concentration non-rebreathing oxygen mask is used to administer pure (100%) oxygen, the actual inhaled oxygen concentration can fall within the region of 60-80%. In practice, there is considerable variation around these values.

There exists a need for a more efficient device for delivering medical gases to a patient, which provides an accurate concentration of the supplied gas.

Patent document WO2021/035306A1 is hereby acknowledged.

### Summary

The invention is defined by the appended claims.

The present specification describes apparatus for delivering gas to a user. The apparatus comprises a patient interface configured to surround the user's mouth and nose. The apparatus also comprises an outer chamber configured to receive gas from an external supply, wherein the outer chamber is secured to a patient facing side of the patient interface and comprises one or more vents, wherein the one or more vents are configured to be closed when the apparatus is secured on the user.

The apparatus further comprises a reservoir in fluid communication with the outer chamber; a connector configured to receive gas flow from the outer chamber, the connector comprising one or more openings to allow an exchange of gas between the connector and the reservoir; an inner chamber in fluid communication with the user's airway; one or more inspiratory valves configured to allow gas to flow from the reservoir into the inner chamber when the gas pressure in the reservoir is higher than the gas pressure in the inner chamber; and an expiratory valve configured to allow gas to flow out of the apparatus when the gas pressure in the inner chamber exceeds the ambient pressure.

In an embodiment, the apparatus is configured such that an external wall of the outer chamber is attached continuously to the patient interface, and an internal wall of the outer chamber is attached to the patient interface at intervals to form the one or more vents. The one or more vents may be configured to be opened when the apparatus is not secured to the user.

The apparatus may further comprise an anti-asphyxia valve connected to the connector, the anti-asphyxia valve being configured to remain closed when the gas pressure in the connector is equal to or exceeds ambient pressure. The anti-asphyxia valve may be configured to open when the gas pressure in the connector is lower than the ambient pressure.

In some embodiments, the volume of the outer chamber is variable, and the outer chamber is configured to inflate upon receiving gas from the external supply while the one or more vents are closed.

The reservoir may be configured to store the gas received from the connector when the one or more inspiratory valves are closed. In some embodiments, the one or more inspiratory valves are configured to open during an expiratory pause. In some embodiments, the one or more inspiratory valves are configured to open when the user inhales. In some embodiments, the expiratory valve is configured to open when the user exhales.

In some embodiments, the patient interface is deformable and/or resilient. For example, the patient interface may comprise memory foam.

The apparatus may additionally comprise one or more straps configured to hold the apparatus in place during use. The one or more straps may be quick release straps.

The apparatus may further comprise a medical gas connector connected to an external gas supply.

### Brief description of the drawings

Figure 1 shows an apparatus according to the present technology;
Figure 2 shows a patient interface according to the present technology;
Figure 3 shows a clip for a strap according to an embodiment of the present technology;
Figure 4 shows an arrangement of straps according to an embodiment of the present technology;
Figure 5 shows an apparatus according to the present technology;
Figure 6 shows a face-on view of a patient interface according to the present technology;
Figure 7 shows a connector and anti-asphyxia valve according to the present technology;
Figure 8 shows one or more inspiratory valves and an expiratory valve according to the present technology;
Figure 9 shows a flowchart depicting the user's respiratory pattern;
Figures 10a and 10b show the results of an experiment using a current industry standard high concentration non-rebreathing oxygen mask and a device according to the present technology.
Figure 11 shows an alternative view of the apparatus of Figure 1.

### Detailed description

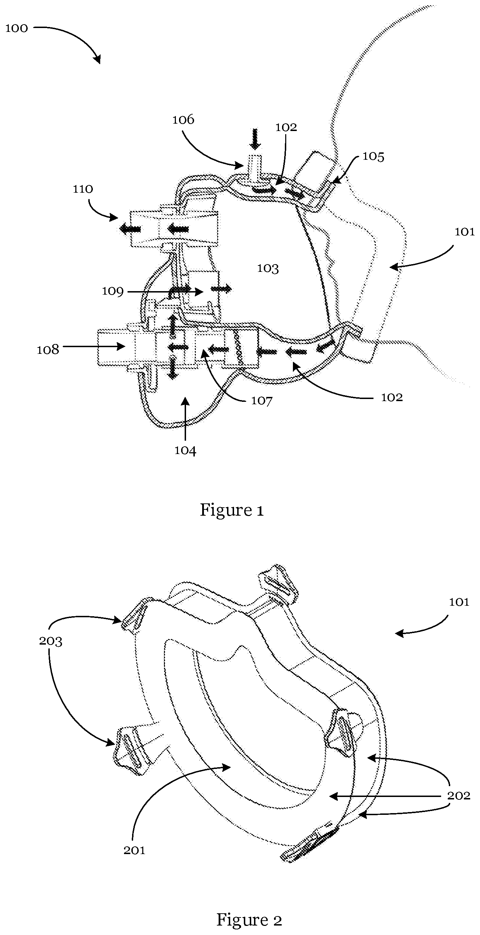

Figure 1 shows a schematic cross section of an apparatus 100 for delivering gas to a user according to the present technology.

The apparatus 100 is designed to deliver gas to a spontaneously breathing human without the application of positive pressure to the airway during inspiration.

The apparatus 100 comprises a patient interface 101 configured to surround the user's mouth and nose. The patient interface 101 may be deformable, such that when secured to the user, the patient interface moulds to fit the contours of the user's face. Additionally or alternatively, the patient interface 101 may be resilient, such that it returns to its original shape when removed from the user. By way of example, the patient interface 101 may comprise a memory foam, such as a polyurethane memory foam. Other suitable materials will be known to the person skilled in the art.

Figure 2 shows an example construction of the patient interface 101. The patient interface 101 comprises a foam layer 201. The patient interface 101 additionally comprises a fabric layer 202 covering three surfaces of the patient interface, the fabric layer 202 being configured to hold the foam layer 201 in position against the user's face. In an example construction, the fabric layer is applied to the patient-facing side of the patient interface 101; the outward-facing side of the patient interface 101; and the outer edge of the patient interface 101, such that the foam layer 201 is exposed at the inner edge of the patient interface. An example of a suitable fabric is a non-woven polypropylene fabric, although any suitable material may be used.

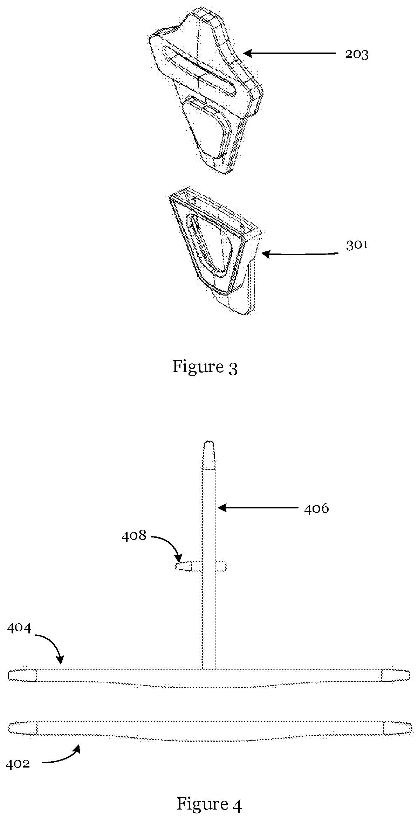

The apparatus 100 is fastened to the user during use. For example, the apparatus 100 may comprise one or more straps (see Fig. 4) configured to hold the apparatus 100 in place during use. As shown in Figure 2, the one or more straps may be attached to the patient interface 101 by one or more clips 203. A closer view of an example clip 203 is shown in Figure 3. The one or more straps may be quick release straps configured to allow the apparatus 100 to be removed quickly from the user, for example in an emergency. For example, the clip 203 may connect to a buckle 301 to allow quick release of the adjoining strap.

An example arrangement of straps 400 is shown in Figure 4. The straps are designed to wrap around the back of the user's head for a secure fit. The straps may comprise a lower strap 402, an upper strap 404 and an overhead strap 406. The overhead strap 406 may also comprise a tube strap 408 configured to retain a gas tube running along the length of the overhead strap 406 when the apparatus 100 is in use. Other methods of securing the apparatus 100 in place during use may also be used.

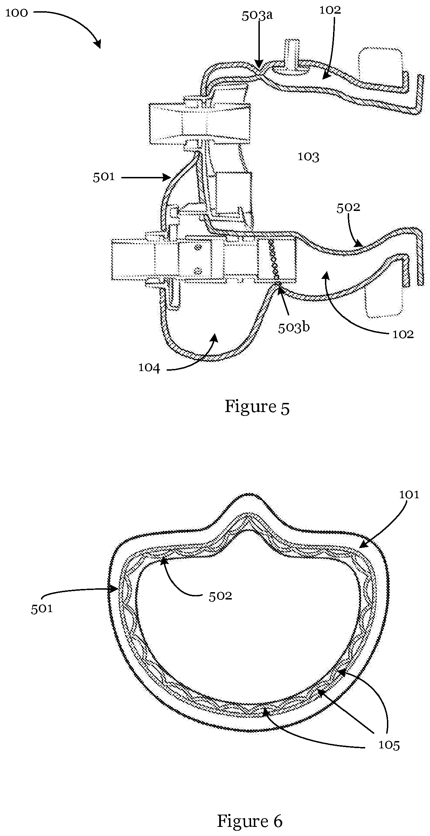

Returning to Figure 1, the apparatus 100 also comprises an outer chamber 102; an inner chamber 103; and a reservoir 104. Figure 5 also shows the arrangement of these chambers within the apparatus 100. The apparatus comprises an outer bag 501 and an inner bag 502. The outer bag 501 is connected to the inner bag 502 at points 503a and 503b. Note that this connection 503 extends around the full circumference of the apparatus 100 to form the outer chamber 102 and reservoir 104. In particular, the inner chamber 103 is formed by the interior of the inner bag 502. The outer chamber 102 is formed between the patient interface 101 and the connection at 503, such that the outer wall of the outer chamber 102 is formed by the outer bag 501 and the inner wall of the outer chamber 102 is formed by the inner bag 502 (external surface). The reservoir 104 is formed on the other side of the connection at 503.

The apparatus also comprises one or more vents 105, as shown in Figure 1. Figure 6 shows a face-on view of the patient interface 101. The outer bag 501 is attached continuously to the patient-facing side of the patient interface 101. The inner bag 502 is attached to the patient-facing side of the patient interface 101 at intervals to form one or more vents 105. The intervals may be evenly spaced, such that each of the one or more vents has the same length. Alternatively, the intervals may be unevenly distributed. The length of each vent may be between 10mm and 20mm.

The one or more vents 105 are configured to be opened when the apparatus 100 is not secured to the user, as shown in Figure 6. When the apparatus 100 is secured to the user, the patient interface 101 moulds to the contours of the user's face as described above, closing the one or more vents 105 to prevent gas leaking out of the outer chamber via the vents. In other words, the outer chamber 102 is formed and sealed by the correct application of the apparatus 100 to the user. In this way, the fit of the patient interface 101 to the user can be tested. If gas leaks out of the vents, the patient interface is not securely moulded to the user to form an air tight seal. This results in a loss of the supplied gas to the external atmosphere. Such a leak may be detected by the user themselves e.g. by sensing the flow of gas over the face, or by a medical professional observing that the outer chamber 102 is not inflating. The fit of the patient interface 101 can then be adjusted accordingly.

When applied correctly to the user, apparatus 100 forms a sealed environment with no inflow of atmospheric air. As a result, the supplied gas is not diluted by atmospheric air as it passes through the apparatus 100. The user only inhales the gas supplied to the device from the external supply. The apparatus 100 thus provides accurate medical gas administration with no variability in administered gas concentrations from breath to breath or within each breath. All ventilated parts of the lungs are exposed to gas of the same concentration, without the application of positive pressure to the airway and thoracic cavity. This mimics the normal physiological situation where the lungs are in equilibrium with atmospheric air, but for the metabolic consumption of oxygen and production of carbon dioxide.

Further, when in use the only gas flowing through the apparatus 100 is the supplied medical gas. Any air present in the apparatus 100 is flushed out by the flow of the supplied gas. If any micro leaks are present in the apparatus, some of this supplied gas flow will leak out of the apparatus 100. However, this 'leak flow' prevents surrounding atmospheric air from entering the apparatus 100 provided an adequate volume and flow of supplied gas is retained within and through apparatus 100 to meet user demand. This is clear from the state of inflation of outer chamber 102 and the state of inflation / deflation of the reservoir 104; hence the concentration of gas inhaled by the user remains constant.

The volume of the outer chamber 102 may be variable. For example, the outer bag 501 may comprise a flexible material. In an embodiment, the outer bag 501 and/or the inner bag 502 are formed of polyethylene or polypropylene.

The outer chamber 102 may be configured to visibly expand upon receiving gas from the external supply, provided the one or more vents 105 are closed. In this way, the fit of the patient interface 101 to the user can be tested, as any leaks would prevent the outer chamber from fully expanding. Correct fitting of the apparatus 100 to the user may also be made immediately apparent to anyone assisting the user and to the user themselves via inflation of the outer chamber 102.

Returning to Figure 1, gas enters into the outer chamber 102 via an external supply. For example, the apparatus 100 may comprise a medical gas connector 106, wherein the medical gas connector 106 is connected to the outer chamber 102. The other end of the medical gas connector 106 is connected to an external gas supply, for example via tubing. The medical gas connector 106 may be a standard connector, such as a 6mm oxygen stem.

The gas may be supplied to the user airway at a pressure consistent with the user's natural respiratory pattern. For example, in normal operation the gas may be supplied to the medical gas connector 106, and hence to the user's airway, at a pressure not exceeding ambient pressure . In other words, there is no positive pressure applied to the user's airway, and the user is able to breathe spontaneously following their natural respiratory pattern.

The supplied gas may comprise any gas to be administered to a user, such as a therapeutic gas. For example, the gas may be pure oxygen, or a mixture of oxygen and another gas. Examples include Nitrox (nitrogen and oxygen), Heliox (helium and oxygen), Argox (argon and oxygen) and Entonox (nitrous oxide and oxygen). In some embodiments, the supplied gas may be augmented with the output of vibrating mesh nebulisers to enable delivery of inhaled drugs.

The apparatus 100 is capable of administering medical gas mixtures of known concentration with accuracy according to clinical need. Because the administration of the inhaled gas mixture is precise, the supplied gas can be titrated to best clinical effect according the latest medical research. This has benefits of improved patient assessment, improved low dependency respiratory support and improved ability to rescue the patient to a clinical area where higher modalities of respiratory support can be introduced.

The gas flows from the outer chamber 102 into the reservoir 104 via a connector 107. The flow of gas is indicated by arrows in Figure 1. The reservoir 104 is configured to store the gas received from the outer chamber 102 when not required by the user. If the fit of the patient interface 101 to the user is correct then no gas flow will be lost and the entire volume of gas delivered into the outer chamber 102 will flow into the reservoir 104, meaning the apparatus 100 is working efficiently.

In some embodiments, the volume of the reservoir 104 may be variable. For example, the reservoir 104 may be configured to visibly expand as gas flows from the outer chamber 102 into the reservoir 104 via the connector 107. Provided the gas flow is adequate for the patient's needs then the reservoir 104 will fill and inflate, giving a visual indicator of correct performance. Failure of the reservoir 104 to expand may indicate a problem with the flow of gas from the outer chamber 102, or that the user's respiratory pattern requires a higher rate of gas flow from the external supply. In this way, direct observation of the apparatus 100 in use informs an attendant medical professional that the device is working correctly, or not, thereby allowing corrective action to be taken. This is further supported by observation of the expansion of the outer chamber 102, as described above. Because correct application to the user is intrinsic to the function of the apparatus 100, the present technology overcomes known issues of prior art devices leaking gas due to poor fitting to the user.

The connector 107 is configured to receive gas flow from the outer chamber 102. The connector 107 comprises one or more openings to allow an exchange of gas between the connector 107 and the reservoir 104. The one or more openings may be evenly or unevenly spaced. For example, the connector 107 may comprise four openings spaced at 90° intervals around the circumference of the connector 107. The connector 107 may take any suitable shape. In some embodiments, the connector 107 is a cylindrical connector.

The apparatus 100 may additionally comprise an anti-asphyxia valve 108 connected to the connector 107. The anti-asphyxia valve 108 is configured to remain closed when the gas pressure in the connector 107 is equal to or exceeds ambient pressure. On the other hand, when the gas pressure in the connector 107 is lower than ambient pressure, the anti-asphyxia valve 108 is configured to open to allow surrounding atmospheric air to flow into the apparatus 100 and enable the user to breathe freely. In this way, the user is protected against a failure in the flow of gas through the apparatus 100, for example if the external supply failed or the flow of supplied gas is inadequate for the user's respiratory pattern.

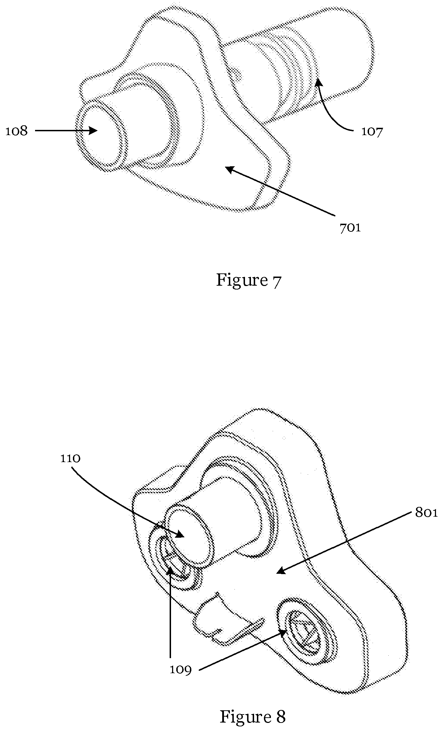

Figure 7 shows an example construction of the connector 107 and the anti-asphyxia valve 108. The connector 107 and the anti-asphyxia valve 108 are attached to a mount 701, which is secured in a gas-tight fashion to the outer bag 501.

The apparatus 100 further comprises an inner chamber 103 in fluid communication with the user's airway. As the user inhales, gas is drawn from the reservoir 104 into the inner chamber 103 via one or more inspiratory valves 109.

The reservoir 104 is configured to store the gas received from the outer chamber 102 when the one or more inspiratory valves 109 are closed. The one or more inspiratory valves 109 are configured to open to allow gas to flow from the reservoir 104 into the inner chamber 103 when the gas pressure in the reservoir 104 is higher than the gas pressure in the inner chamber 103. For example, the one or more inspiratory valves 109 may be configured to open during an expiratory pause and/or when the user inhales. As the user exhales, the gas flows out of the apparatus 100 via an expiratory valve 110. The expiratory valve 110 is configured to open to allow gas to flow out of the apparatus 100 when the gas pressure in the inner chamber 103 exceeds the ambient pressure. For example, the expiratory valve 110 may be configured to open when the user exhales.

Figure 8 shows an example construction of the inspiratory valves 109 and the expiratory valve 110. The inspiratory valves 109 and the expiratory valve 110 are attached to a mount 801, which is secured in a gas-tight fashion to the inner bag 502. The outer bag 501 is secured in a gas tight fashion around the external edge of the expiratory valve 110.

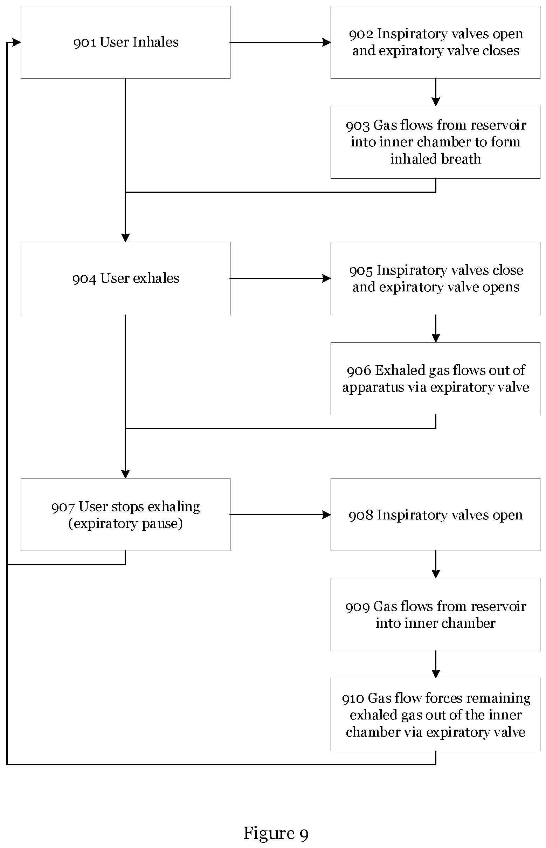

By way of example, consider the respiratory pattern of the user. Figure 9 is a flowchart detailing the flow of gas through the apparatus during the respiratory pattern of the user. The steps on the left hand side of the chart show actions of the user, while the steps on the right hand side show operations of the apparatus. At 901 the user inhales. As the user inhales, the pressure in the inner chamber 103 decreases, such that the gas pressure in the inner chamber is lower than both the gas pressure in the reservoir 104 and the ambient pressure. As a result, at 902 the one or more inspiratory valves 109 open and the expiratory valve 110 closes. At 903 gas is drawn from the reservoir 104 into the inner chamber 103 to form the user's inhaled breath.

At 904 the user exhales. As the user exhales, the pressure in the inner chamber 103 increases, such that the gas pressure in the inner chamber exceeds both the gas pressure in the reservoir 104 and the ambient pressure. As a result, at 905 the one or more inspiratory valves 109 close and the expiratory valve 110 opens. At 906 the exhaled gas flows out of the apparatus 100 via the expiratory valve 110.

At 907 the user stops exhaling (an expiratory pause). Once the user stops exhaling, at 908 the one or more inspiratory valves 109 open, and gas flows from the reservoir 104 into the inner chamber 103 (909). This gas flow is sufficient to prevent the expiratory valve from closing. The pressure inside the inner chamber 103 may be slightly positive after the user has exhaled and the inspiratory valves 109 have opened to allow gas into the inner chamber 103. In an embodiment, the pressure in the inner chamber 103 does not exceed 981 Pa above ambient pressure.

At 910, the flow of gas forces any remaining exhaled gas out of the inner chamber 103 via the expiratory valve 110, thus venting the inner chamber 103 prior to the next inhalation. This ensures the user's next inhalation contains only the supplied gas and not any exhaled gas, in particular exhaled carbon dioxide. The pressure in the inner chamber 103 will be dependent on the flow going into inner chamber 103 and thence out through the expiratory valve 110. The pressure in the inner chamber 103 will therefore be determined by the flow resistance of the expiratory valve 110.

In normal operation, once the user inhales, the pressure in the inner chamber 103 will fall to below ambient pressure, provided the gas is supplied at a pressure consistent with the user's respiratory pattern. The inner chamber 103 thus collapses during inspiration.

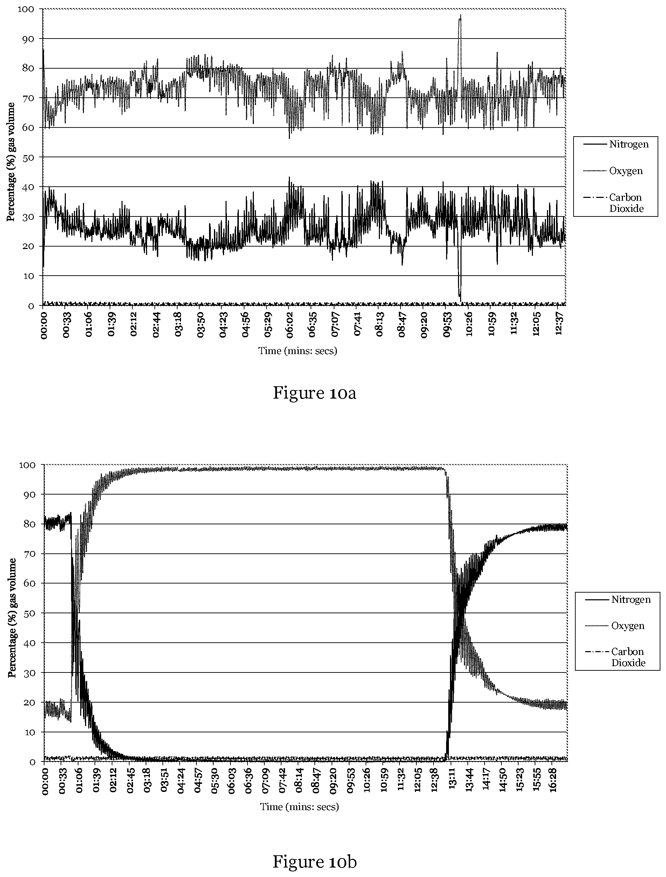

The effect of this design is to ensure that titration of the gas flow to the user's respiratory need is accurate, which in turn makes the mask efficient. Figure 10a shows the results of an experiment using a current industry standard high concentration non-rebreathing oxygen mask. In particular, Figure 10a shows the composition of gas received by the user over time, whilst using a current industry standard high concentration non-rebreathing oxygen mask, as a percentage of the total gas volume received by the user. The mask is supplied with 100% oxygen. It is apparent from Figure 10a that the user only receives around 70% oxygen, with the remaining breath made up of nitrogen from the ambient air. Thus, only a fraction of the supplied gas is actually received by the user.

Figure 10b shows the same experiment, but this time performed using a device according to the present technology.

The experiment was performed under the same conditions as for the current industry standard high concentration non-rebreathing oxygen mask. Initially, the user breathes ambient air (78% nitrogen, 21% oxygen, trace carbon dioxide). At 1 minute the gas supply of 100% oxygen is turned on, and the composition gas received by the user quickly equalizes at close to 100% oxygen, with trace amounts of nitrogen. At 13 minutes the gas supply is removed, and the user returns to breathing ambient air. Thus, when a device according to the present technology is used, close to 100% of the supplied gas is received by the user. It is clear that the present device provides significantly more accurate titration of the gas flow to the user's respiratory need.

Apparatus according to the present technology requires considerably less gas flow compared to prior art hood devices described above (approximately 1.5x patient minute volume for the present technology compared to 5x patient minute volume for a conventional hood device).

Returning to Figure 1, the expiratory valve 110 may additionally comprise a filter, configured to filter the exhaled gas as it flows out of the apparatus 100. Because the patient interface 101 moulds to the user's face to close the one or more vents 105, the user's respiratory tract is isolated, such that all exhaled gas must pass through the expiratory valve. Hence, providing a filter in the expiratory valve 110 prevents onward transmission of airborne micro-organisms which may be present in the exhaled gas. This may also be used for microbiological sampling and capture of exhaled microbes. Subsequent analysis using modern molecular diagnostic techniques for pathogenic microbes may improve rapid diagnostic yields and subsequent clinical decisions regarding therapy.

The effective isolation of the respiratory tract provided by the apparatus 100 has applications where there is concern about transmission of an airborne pathogen. In addition to delivering medical gases to a user, the apparatus 100 could alternatively be used by professionals working in high risk environments. In such case, the apparatus 100 could be connected to a source of filtered atmospheric air.

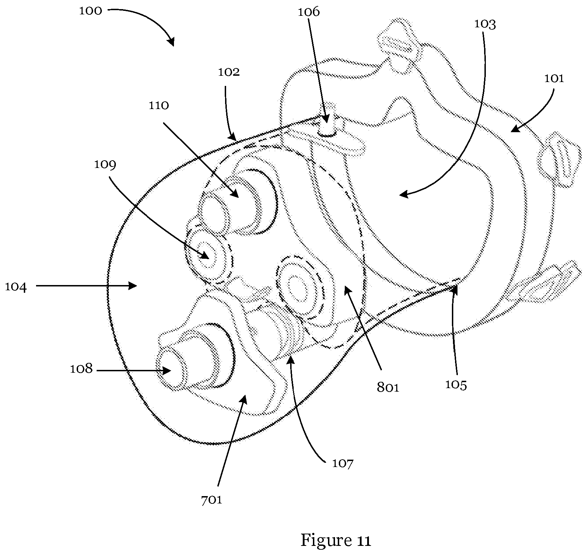

Figure 11 shows the apparatus of Figure 1 from a different angle.

The apparatus 100 is simple to use and lightweight. It could be used by any appropriately trained user, including directly by a patient themselves, enabling management of their own condition. This would be particularly advantageous if the apparatus 100 were used to administer inhaled drugs, for example by using a helium/oxygen gas mixture to aid the delivery of nebulised drugs into the lung.

Various modifications and variations will be apparent to those skilled in the art which fall within the scope of the following claims.

## Claims

1. Apparatus (100) for delivering gas to a user without the application of positive pressure, comprising:
a patient interface (101) configured to surround the user's mouth and nose;
an outer chamber (102) configured to receive gas from an external supply, wherein the outer chamber is secured to a patient facing side of the patient interface and comprises one or more vents (105), wherein the one or more vents are configured to be closed when the apparatus is secured on the user;
a reservoir (104) in fluid communication with the outer chamber;
a connector (107) configured to receive gas flow from the outer chamber, the connector comprising one or more openings to allow an exchange of gas between the connector and the reservoir;
an inner chamber (103) in fluid communication with the user's airway;
one or more inspiratory valves (109) configured to allow gas to flow from the reservoir into the inner chamber when the gas pressure in the reservoir is higher than the gas pressure in the inner chamber; and
an expiratory valve (110) configured to allow gas to flow out of the apparatus when the gas pressure in the inner chamber exceeds the ambient pressure.

2. The apparatus of claim 1, wherein an external wall of the outer chamber is attached continuously to the patient interface, and an internal wall of the outer chamber is attached to the patient interface at intervals to form the one or more vents.

3. The apparatus of any preceding claim, wherein the one or more vents are configured to be opened when the apparatus is not secured to the user.

4. The apparatus of any preceding claim, further comprising an anti-asphyxia valve (108) connected to the connector, the anti-asphyxia valve being configured to remain closed when the gas pressure in the connector is equal to or exceeds ambient pressure.

5. The apparatus of claim 4, the anti-asphyxia valve being configured to open when the gas pressure in the connector is lower than the ambient pressure.

6. The apparatus of any preceding claim, wherein the volume of the outer chamber is variable and wherein the outer chamber is configured to inflate upon receiving gas from the external supply while the one or more vents are closed.

7. The apparatus of any preceding claim, wherein the reservoir is configured to store the gas received from the connector when the one or more inspiratory valves are closed.

8. The apparatus of any preceding claim, wherein the one or more inspiratory valves are configured to open during an expiratory pause.

9. The apparatus of any preceding claim, wherein the one or more inspiratory valves are configured to open when the user inhales.

10. The apparatus of any preceding claim, wherein the expiratory valve is configured to open when the user exhales.

11. The apparatus of any preceding claim, wherein the patient interface is deformable.

12. The apparatus of claim 11, wherein the patient interface is resilient.

13. The apparatus of claim 11 or 12, wherein the patient interface comprises memory foam.

14. The apparatus of any preceding claim, further comprising one or more straps configured to hold the apparatus in place during use, wherein optionally the one or more straps are quick release straps.

15. The apparatus of any preceding claim, further comprising a medical gas connector (106) connected to an external gas supply.

## Patentansprüche

1. Vorrichtung (100) zum Liefern von Gas an einen Benutzer ohne die Anwendung von Überdruck, umfassend:
eine Patientenschnittstelle (101), die dazu konfiguriert ist, den Mund und die Nase des Benutzers zu umgeben;
eine Außenkammer (102), die dazu konfiguriert ist, Gas von einer externen Versorgung zu empfangen, wobei die Außenkammer an einer dem Patienten zugewandten Seite der Patientenschnittstelle gesichert ist und eine oder mehrere Entlüftungen (105) umfasst, wobei die eine oder mehreren Entlüftungen dazu konfiguriert sind, geschlossen zu sein, wenn die Vorrichtung an dem Benutzer gesichert ist;
einen Behälter (104) in Fluidkommunikation mit der Außenkammer;
einen Verbinder (107), der dazu konfiguriert ist, Gasstrom aus der Außenkammer zu empfangen, wobei der Verbinder eine oder mehrere Öffnungen umfasst, um einen Gasaustausch zwischen dem Verbinder und dem Behälter zu ermöglichen;
eine Innenkammer (103) in Fluidkommunikation mit den Atemwegen des Benutzers;
ein oder mehrere Inspirationsventile (109), die dazu konfiguriert sind, zu ermöglichen, dass Gas aus dem Behälter in die Innenkammer strömt, wenn der Gasdruck in dem Behälter höher als der Gasdruck in der Innenkammer ist; und
ein Exspirationsventil (110), das dazu konfiguriert ist, zu ermöglichen, dass Gas aus der Vorrichtung strömt, wenn der Gasdruck in der Innenkammer den Umgebungsdruck übersteigt.

2. Vorrichtung nach Anspruch 1, wobei eine externe Wand der Außenkammer durchgehend an der Patientenschnittstelle angebracht ist und eine interne Wand der Außenkammer an der Patientenschnittstelle in Intervallen angebracht ist, um die eine oder mehreren Entlüftungen zu bilden.

3. Vorrichtung nach einem vorhergehenden Anspruch, wobei die eine oder mehreren Entlüftungen dazu konfiguriert sind, geöffnet zu sein, wenn die Vorrichtung nicht an dem Benutzer gesichert ist.

4. Vorrichtung nach einem vorhergehenden Anspruch, ferner umfassend ein Anti-Asphyxie-Ventil (108), das mit dem Verbinder verbunden ist, wobei das Anti-Asphyxie-Ventil dazu konfiguriert ist, geschlossen zu bleiben, wenn der Gasdruck in dem Verbinder gleich dem Umgebungsdruck ist oder diesen übersteigt.

5. Vorrichtung nach Anspruch 4, wobei das Anti-Asphyxie-Ventil dazu konfiguriert ist, sich zu öffnen, wenn der Gasdruck in dem Verbinder niedriger als der Umgebungsdruck ist.

6. Vorrichtung nach einem vorhergehenden Anspruch, wobei das Volumen der Außenkammer variabel ist und wobei die Außenkammer dazu konfiguriert ist, sich beim Empfangen von Gas von der externen Versorgung aufzublasen, während die eine oder mehreren Entlüftungen geschlossen sind.

7. Vorrichtung nach einem vorhergehenden Anspruch, wobei der Behälter dazu konfiguriert ist, das Gas, das von dem Verbinder empfangen wird, zu speichern, wenn das eine oder die mehreren Inspirationsventile geschlossen sind.

8. Vorrichtung nach einem vorhergehenden Anspruch, wobei das eine oder die mehreren Inspirationsventile dazu konfiguriert sind, sich während einer Exspirationspause zu öffnen.

9. Vorrichtung nach einem vorhergehenden Anspruch, wobei das eine oder die mehreren Inspirationsventile dazu konfiguriert sind, sich zu öffnen, wenn der Benutzer inhaliert.

10. Vorrichtung nach einem vorhergehenden Anspruch, wobei das Exspirationsventil dazu konfiguriert ist, sich zu öffnen, wenn der Benutzer exhaliert.

11. Vorrichtung nach einem vorhergehenden Anspruch, wobei die Patientenschnittstelle verformbar ist.

12. Vorrichtung nach Anspruch 11, wobei die Patientenschnittstelle elastisch ist.

13. Vorrichtung nach Anspruch 11 oder 12, wobei die Patientenschnittstelle Memory-Schaum umfasst.

14. Vorrichtung nach einem vorhergehenden Anspruch, ferner umfassend einen oder mehrere Gurte, die dazu konfiguriert sind, die Vorrichtung während Verwendung an Ort und Stelle zu halten, wobei optional der eine oder die mehreren Gurte Schnelllösgurte sind.

15. Vorrichtung nach einem vorhergehenden Anspruch, ferner umfassend einen medizinischen Gasverbinder (106), der mit einer externen Gasversorgung verbunden ist.

## Revendications

1. Appareil (100) permettant d'administrer un gaz à un utilisateur sans application de pression positive, comprenant :
une interface patient (101) conçue pour entourer la bouche et le nez de l'utilisateur ;
une chambre externe (102) conçue pour recevoir du gaz en provenance d'une alimentation externe, dans lequel la chambre externe est fixée à un côté faisant face au patient de l'interface patient et comprend un ou plusieurs évents (105), dans lequel l'un ou plusieurs évents sont conçus pour être fermés lorsque l'appareil est fixé à l'utilisateur ;
un réservoir (104) en communication fluidique avec la chambre extérieure ;
un connecteur (107) configuré pour recevoir un flux de gaz provenant de la chambre externe, le connecteur comprenant une ou plusieurs ouvertures pour permettre un échange de gaz entre le connecteur et le réservoir ;
une chambre interne (103) en communication fluidique avec les voies respiratoires de l'utilisateur ;
une ou plusieurs valves inspiratoires (109) conçues pour permettre au gaz de s'écouler du réservoir vers la chambre interne lorsque la pression de gaz dans le réservoir est supérieure à la pression de gaz dans la chambre interne ; et
une valve expiratoire (110) conçue pour permettre au gaz de s'écouler hors de l'appareil lorsque la pression de gaz dans la chambre interne dépasse la pression ambiante.

2. Appareil selon la revendication 1, dans lequel une paroi externe de la chambre externe est attachée en continu à l'interface patient, et une paroi interne de la chambre externe est attachée à l'interface patient à des intervalles pour former l'un ou plusieurs évents.

3. Appareil selon l'une quelconque des revendications précédentes, dans lequel l'un ou plusieurs évents sont conçus pour être ouverts lorsque l'appareil n'est pas fixé à l'utilisateur.

4. Appareil selon l'une quelconque des revendications précédentes, comprenant en outre une valve anti-asphyxie (108) connectée au connecteur, la valve anti-asphyxie étant conçue pour rester fermée lorsque la pression de gaz dans le connecteur est égale ou supérieure à la pression ambiante.

5. Appareil selon la revendication 4, la valve anti-asphyxie étant conçue pour s'ouvrir lorsque la pression de gaz dans le connecteur est inférieure à la pression ambiante.

6. Appareil selon l'une quelconque des revendications précédentes, dans lequel le volume de la chambre externe est variable et dans lequel la chambre externe est conçue pour se gonfler lors de la réception de gaz en provenance de l'alimentation externe alors que l'un ou plusieurs évents sont fermés.

7. Appareil selon l'une quelconque des revendications précédentes, dans lequel le réservoir est conçu pour stocker le gaz reçu du connecteur lorsque l'une ou plusieurs valves inspiratoires sont fermées.

8. Appareil selon l'une quelconque des revendications précédentes, dans lequel l'une ou plusieurs valves inspiratoires sont conçues pour s'ouvrir pendant une pause expiratoire.

9. Appareil selon l'une quelconque des revendications précédentes, dans lequel l'une ou plusieurs valves inspiratoires sont conçues pour s'ouvrir lorsque l'utilisateur inhale.

10. Appareil selon l'une quelconque des revendications précédentes, dans lequel la valve expiratoire est conçue pour s'ouvrir lorsque l'utilisateur expire.

11. Appareil selon l'une quelconque des revendications précédentes, dans lequel l'interface patient est déformable.

12. Appareil selon la revendication 11, dans lequel l'interface patient est élastique.

13. Appareil selon la revendication 11 ou 12, dans lequel l'interface patient comprend de la mousse à mémoire de forme.

14. Appareil selon l'une quelconque des revendications précédentes, comprenant en outre une ou plusieurs sangles conçues pour maintenir l'appareil en place pendant l'utilisation, dans lequel éventuellement l'une ou plusieurs sangles sont des sangles à libération rapide.

15. Appareil selon l'une quelconque des revendications précédentes, comprenant en outre un connecteur de gaz médical (106) connecté à une alimentation en gaz externe.
